# EUROPEAN PATENT SPECIFICATION

(11) **EP 3 822 359 B1**
(45) Date of publication and mention of the grant of the patent: **07.08.2024**
(21) Application number: 19833189.4
(22) Date of filing: 12.07.2019
(51) Int. Cl.: C12Q 1/04, G01N 21/64, G01N 33/48, G01N 33/53, G01N 33/574, G01N 33/58

(54) **PRETREATMENT METHOD FOR FLUORESCENT IMAGE DIAGNOSIS**
VORBEHANDLUNGSVERFAHREN FÜR FLUORESZENZBILDDIAGNOSE
PROCÉDÉ DE PRÉTRAITEMENT POUR DIAGNOSTIC PAR IMAGE DE FLUORESCENCE

(30) Priority: 13.07.2018 JP 2018132928
(43) Date of publication of application: 19.05.2021
(60) Divisional application: 24183773.1
(73) Proprietor: Goryo Chemical, Inc., Sapporo-shi, Hokkaido 060-0008 (JP); Hamamatsu Photonics K.K., Hamamatsu-shi, Shizuoka 435-8558 (JP)
(72) Inventor: MINOURA, Itsushi, Sapporo-shi, Hokkaido 060-0008 (JP); TEZUKA, Yoshiaki, Tokyo 103-0023 (JP); HIRAWAKE, Kazumasa, Hamamatsu-shi, Shizuoka 435-8558 (JP)
(74) Representative: J A Kemp LLP
(86) International application number: PCT/JP2019/027662
(87) International publication number: WO 2020/013308

(56) References cited:
- WO-A1-2016/073941
- US-A1- 2015 299 663
- MILLER F ET AL: "Application of immunologic probes for contractile proteins to tissue sections", CLINICAL IMMUNOLOGY AND IMMUNOPATHOLOGY, SAN DIEGO, CA, US, vol. 5, no. 3, 1 May 1976 (1976-05-01), pages 416 - 428, XP026272173, ISSN: 0090-1229, [retrieved on 19760501], DOI: 10.1016/0090-1229(76)90051-9
- YUKO NAKAMURA ET AL: "Dynamic fluorescent imaging with the activatable probe, [gamma]-glutamyl hydroxymethyl rhodamine green in the detection of peritoneal cancer metastases: Overcoming the problem of dilution when using a sprayable optical probe", ONCOTARGET, vol. 7, no. 32, 9 August 2016 (2016-08-09), XP055580144, DOI: 10.18632/oncotarget.9898
- GRONDAHL, TOR 0 ET AL.: "Depletion of intracellular Ca2+ stores or lowering extracellular calcium alters intracellular Ca2+ changes during cerebral energy deprivation", BRAIN RESEARCH, vol. 796, no. 1-2, 15 June 1998 (1998-06-15), pages 125 - 131, XP055674788

## Description

### Technical Field

The present invention relates to a method of pretreating tissue for fluorescent image diagnosis using a fluorescent diagnostic drug which emits fluorescence on reacting with a tumor marker or the like.

### Background Art

The success of cancer surgery depends on early and accurate localization of cancer cells and complete removal thereof. Hence, the visualization of a tumor region during surgery has been demanded, and fluorescent probes specifically reacting with tumor have been developed (Non Patent Literatures 1 to 8). However, there are problems in simple application of the fluorescent probe directly to a tumor region, such as insufficient fluorescence intensity in a short time period; difficulties in discriminating between the fluorescence and the intrinsic autoluminescence of cells; and rapid disappearance of the fluorescence. Hence, several methods have been studied such as locating a cancer region using a kinetic map (Non Patent Literature 9), cutting, pressing and rinsing treatments (Non Patent Literature 10), a multispectral open-air method (Non Patent Literature 11), and a fluorescent probe cross-linking to an intracellular protein (Non Patent Literature 12).

Patent literature 1 discloses methods and kits for clearing and optionally subsequently visualizing tissue containing bone. Non-patent literature 13 discloses the application of immunologic probes for contractile proteins to tissue sections. Patent literature 2 discloses a method for culturing a subpopulation of circulating epithelial tumor cells from a body fluid of a human or animal suffering from an epithelial tumor.

### Citation List

### Non Patent Literature

Non Patent Literature 1: Yasuteru Urano et al., Science Translational Medicine; 3 (110): 110-119 (2011)
Non Patent Literature 2: Yoichi Miyata et al., Scientific Reports; 7: 36542 (2017)
Non Patent Literature 3: Takeshi Mizushima et al., BMC Cancer; 16: 411 (2016)
Non Patent Literature 4: Yasuteru Urano, Current Opinion in Chemical Biology; 16: 602-608 (2012)
Non Patent Literature 5: Makoto Mitsunaga et al., Gut; 62: 1179-1186 (2013)
Non Patent Literature 6: Hiroaki Ueo et al., Scientific Reports; 5: 12080 (2015)
Non Patent Literature 7: Yoshiaki Shinden et al., Scientific Reports; 6: 27525 (2016) Non Patent Literature 8: Haruaki Hino et al., Translational Oncology; 9: 203-210 (2016)
Non Patent Literature 9: Yuko Nakamura et al., Oncotarget; 7 (32): 51124-51137 (2016)
Non Patent Literature 10: Toshiko Harada et al., Contrast Media Mo. Imaging (2016) DOI: 10.1002/cmmi.1775
Non Patent Literature 11: Ali Behrooz et al., Optics Letters; 42 (15): 2964-2967 (2017) Non Patent Literature 12: Yuko Nakamura et al., Oncotarget; 8 (24): 39512-39521 (2017)
Non Patent Literature 13: Miller et al., Clinical Immunology and Immunopathology, 5(3): 416-428 (1976)

### Patent Literature

Patent Literature 1: WO 2016/073941
Patent Literature 2: US 2015/299663

### Summary of Invention

### Technical Problem

The inventors have studied on the staining of cell masses or tissues using fluorescent probes, and found that there are cases in which fluorescence cannot be observed in contact with a fluorescent probe that is enough to discriminate between a clinically excised tissue or a lymph node and non-cancer regions, although the tissue or the lymph node evidently has cancer. Further study by the inventor revealed that when a cancer cell mass contacts to a fluorescent probe, surface cancer cells rapidly emit fluorescence, whereas internal cancer cells need time for light emission. From these findings, the inventors have newly found the problem that a previous method for detecting cancer cells using a fluorescent probe can hardly detect cancer cells present in regions other than the surface of cell masses. Accordingly, an object of the present invention is to provide a method for enabling a target cell of a fluorescent probe present inside of a cell mass to rapidly emit fluorescence.

### Solution to Problem

The inventors have tried various treatment of a cell mass comprising target cells which is expected to emit fluorescence with a fluorescent probe, and investigated an improvement in light emission. As a result, the inventors found that removal of calcium ion from a cell mass containing target cells can allow the target cells locating inside of the cell mass to emit light rapidly, and thus achieved the present invention. The method of the present invention is a general-purpose method applicable to any adherent cell.

Accordingly, the present invention provides:
(1) A method for emitting fluorescence that can distinguish a target cell from non-target cells, comprising:
   removing calcium ions from within a cell mass or a tissue containing the target cell; and
   contacting the cell mass or the tissue with a fluorescent probe which emits fluorescence when contacted with or taken up into the target cell, and
   enabling the cell mass or the tissue from which the calcium ion was removed and which was contacted with the fluorescent probe to emit fluorescence,
   wherein the fluorescent probe that emits fluorescence when contacted with or taken up into the target cell is a fluorescent probe that emits fluorescence when reacted with an enzyme or iron ions contained in the target cell,
   wherein the removal of the calcium ion is performed by impregnating the cell mass or the tissue containing the target cell with a buffer solution containing a metal ion chelating agent or a buffer solution containing no calcium,
   wherein the method is performed *ex vivo*
(2) The method of (1), which is a method for emitting fluorescence from the inside of a target cell mass, or a method for allowing target cells present inside of a cell mass or a tissue to emit fluorescence.
(3) A method for determining a region of presence of target cells, comprising:
   removing a calcium ion from within a cell mass or a tissue which is expected to contain the target cells;
   contacting the cell mass or the tissue expected to contain the target cells with a fluorescent probe that emits fluorescence when contacted with or taken up into the target cell; and
   determining a region where fluorescence emission from the fluorescent probe is observed, as the region of presence of the target cells,
   wherein the fluorescent probe that emits fluorescence when contacted with or taken up into the target cell is a fluorescent probe that emits fluorescence when reacted with an enzyme or iron ions contained in the target cell,
   wherein the removal of the calcium ion is performed by impregnating the cell mass or the tissue containing the target cell with a buffer solution containing a metal ion chelating agent or a buffer solution containing no calcium,
   wherein the method is performed *ex vivo.*
(4) The method of (1), wherein the enzyme is glycosidase, protease, or peptidase.
(5) The method of (4), wherein the enzyme is an enzyme present on cell membrane surface.
(6) The method of any one of (1)-(5), wherein the impregnation is performed for 30 seconds to 30 minutes.
(7) The method of (1), wherein the fluorescent probe emits fluorescence when contacted with or taken up into the target cell,
   wherein the target cell is a cancer cell or a tumor cell.
(8) The method of (7), wherein the cancer is solid cancer.
(9) The method of any one of (1) to (8), wherein the fluorescent probe is gGlu-HMRG, Leu-HMRG, EP-HMRG or FeRhoNox-1.
(10) A method for diagnosing cancer or tumor in a subject, comprising:
   removing a calcium ion from within a cell mass sample or a tissue sample obtained from a subject;
   contacting the sample with a fluorescent probe that emits fluorescence when contacted with or taken up into a cancer cell or a tumor cell; and
   diagnosing the subject as having cancer or tumor when a cell emitting fluorescence is present in the sample,
   wherein the fluorescent probe that emits fluorescence when contacted with or taken up into the target cell is a fluorescent probe that emits fluorescence when reacted with an enzyme or iron ions contained in the target cell,
   wherein the removal of the calcium ion is performed by impregnating the cell mass or the tissue containing the target cell with a buffer solution containing a metal ion chelating agent or a buffer solution containing no calcium,
   wherein the method is performed *ex vivo.*

### Advantageous Effects of Invention

The method for allowing a target cell to emit fluorescence according to the present invention can let the target cell present inside a cell mass or a tissue emit light, and thus can be used in the detection of a target cell in a sample, such as a clinical sample, in which the target cell may not be present on the surface.

### Brief Description of Drawings

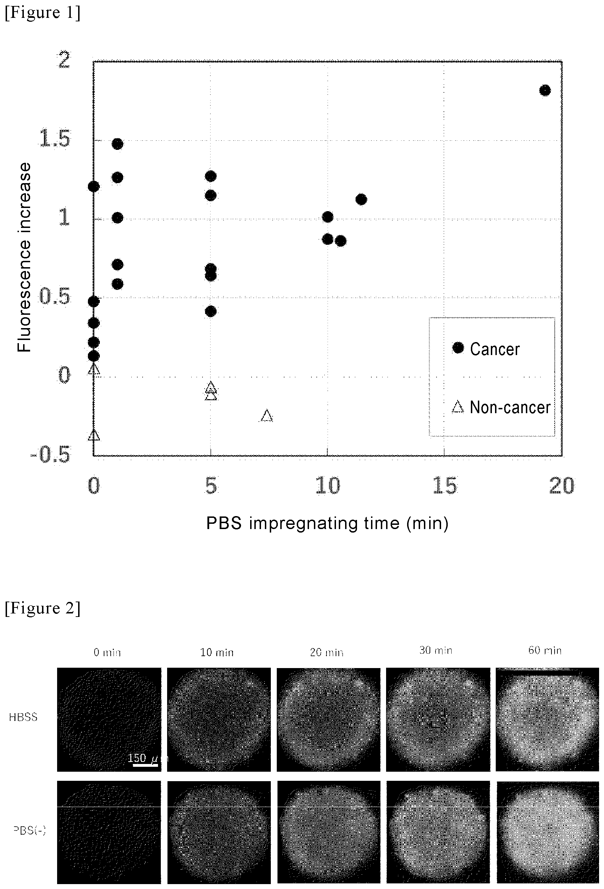

[Figure 1] Figure 1 shows the effect of PBS(-) pretreatment of clinical cancer specimens. After pretreatment of lymph node specimens resected during breast cancer surgery in PBS(-) for 0 minutes, 1 minute, 5 minutes, 10 minutes or an appropriate course of time, increases of fluorescence at 10 minutes after impregnation with a 50 µM gGlu-HMRG solution from that immediately after the impregnation are shown. A specimen diagnosed with cancer metastasis in pathological diagnosis is indicated by a filled circle, and a specimen confirmed to have no cancer metastasis is indicated by an open triangle.
[Figure 2] Figure 2 shows the effect of PBS(-) pretreatment of cancer-derived cultured cell masses (spheroids). Time-dependent changes in fluorescence intensity are shown for a HeLa cells spheroid which was washed with HBSS and then impregnated with a 1 µM solution of gGlu-HMRG dissolved in HBSS (upper), and for a HeLa cells spheroid which was impregnated with PBS(-) for 10 minutes and then impregnated with a 1 µM solution of gGlu-HMRG dissolved in PBS(-) (lower).

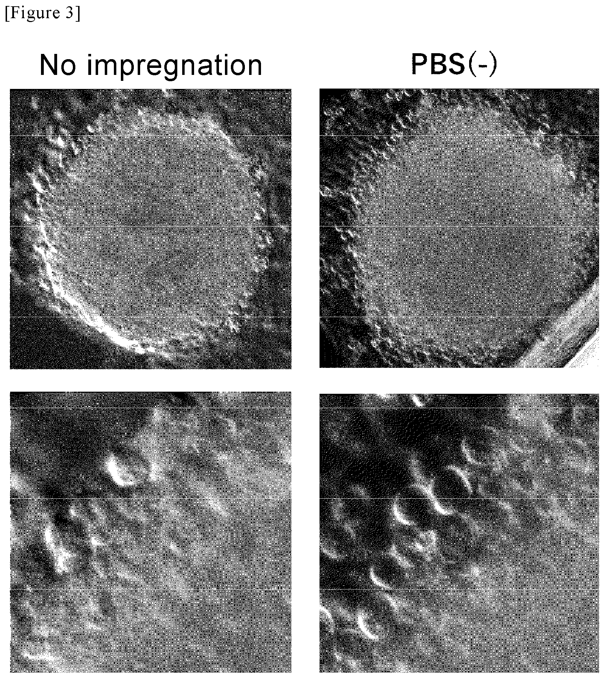

[Figure 3] Figure 3 shows the influence of PBS(-) on cell adhesion. Transmitted light images of cells taken immediately after impregnating a spheroid with a 1 µM gGlu-HMRG solution are shown. The spheroid was not pretreated with PBS(-) (left) or pretreated by impregnation with PBS(-) for 10 minutes. The upper pictures show whole images, and the lower pictures show enlarged images of the marginal regions of the cell masses.

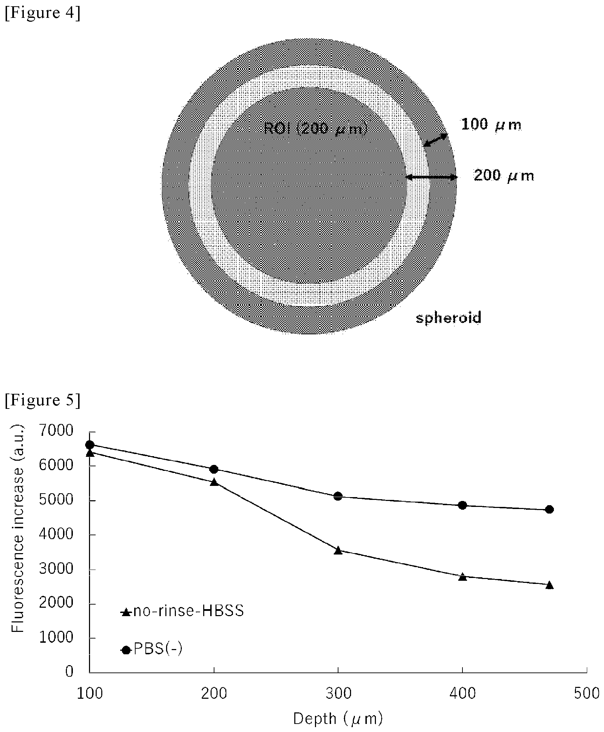

[Figure 4] Figure 4 illustrates a method for analyzing the penetration or reaction of reagents into or with the spheroid. When "Depth" shown in Figures 5 to 10 was, for example, 200 µm, the fluorescence intensity of an open region in the illustration was determined from fluorescence intensity observed by fluorescence microscope, wherein the open region has the inside circle at 200 µm inside from the outer periphery and has the outside circle at 100 µm outside from the aforementioned inside circle as shown in the drawing.
[Figure 5] Figure 5 shows change in the penetration and reaction of a reagent into or with a spheroid by PBS(-) pretreatment. Fluorescence intensity distributions of a non-pretreated HeLa cell spheroid impregnated with a 50 µM solution of gGlu-HMRG dissolved in HBSS for 5 minutes (filled triangle) and of a HeLa cell spheroid which is pretreated by impregnating the spheroid with PBS(-) for 10 minutes, and then impregnated with a 50 µM solution of gGlu-HMRG dissolved in PBS(-) for 5 minutes (filled circle).

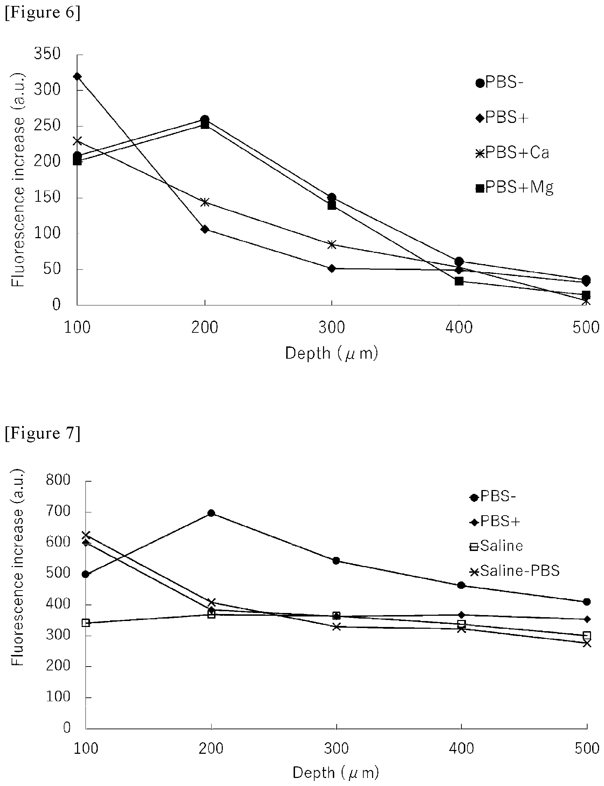

[Figure 6] Figure 6 shows difference in the pretreatment effect for each pretreatment solution composition. Fluorescence intensity distributions of an A549 cell spheroid which was impregnated with each solution of PBS- (filled circle), PBS+ (filled rhombus), PBS+Ca (*), or PBS+Mg (filled square) for 10 minutes and then impregnated with a 50 µM solution of gGlu-HMRG dissolved in each solution for 5 minutes.
[Figure 7] Figure 7 shows difference in the pretreatment effect for each pretreatment solution composition. Fluorescence intensity distributions of an A549 cell spheroid which was impregnated with PBS- (filled circle), PBS+ (filled rhombus), or saline (open square, *) for 10 minutes and then impregnated with a 50 µM solution of gGlu-HMRG dissolved in each solution of PBS- (filled circle, *), PBS+ (filled rhombus), or saline for 5 minutes (open square).

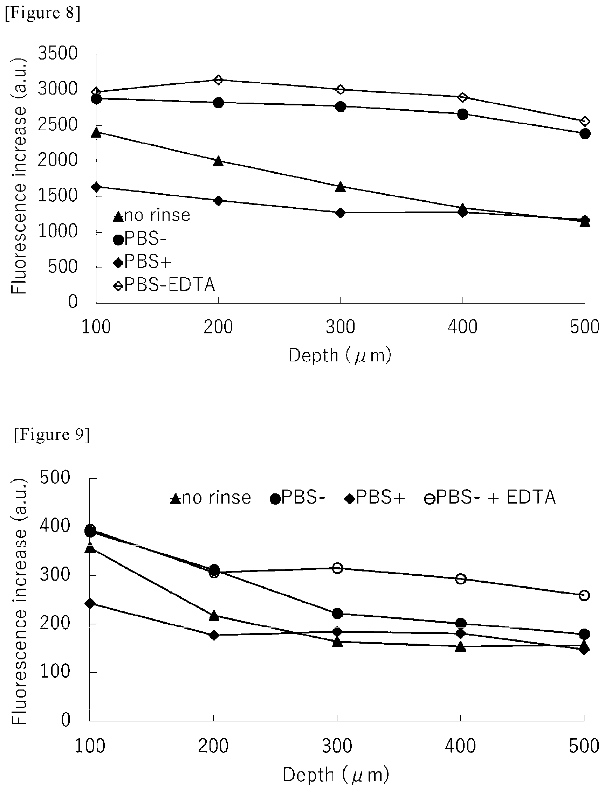

[Figure 8] Figure 8 shows difference in the penetration/reaction of a reagent depending on the presence or absence of pretreatment and the pretreatment solution composition. Fluorescence intensity distributions of a non-pretreated HeLa cell spheroid (filled triangle), and a HeLa cell spheroid which was impregnated with each solution of PBS-(filled circle), PBS+ (filled rhombus), or PBS-EDTA (open rhombus) for 10 minutes, which were impregnated with a 50 µM solution of gGlu-HMRG dissolved in each solution of PBS- (filled triangle, filled circle), PBS+ (filled rhombus), or PBS-EDTA (open rhombus) for 5 minutes.
[Figure 9] Figure 9 shows difference in the penetration or reaction of a reagent depending on the presence or absence of pretreatment and the pretreatment solution composition. Fluorescence intensity distributions of a non-pretreated A549 cell spheroid (filled triangle), and an A549 cell spheroid impregnated with each solution of PBS- (filled circle), PBS+ (filled rhombus), or PBS-EDTA (open rhombus) for 10 minutes, which is impregnated with a 50 µM solution of gGlu-HMRG dissolved in each solution for 5 minutes of, PBS- (filled triangle, filled circle), PBS+ (filled rhombus), or PBS-EDTA (open rhombus).

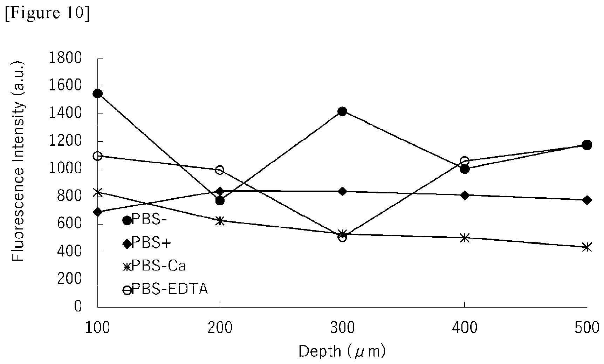

[Figure 10] Figure 10 shows difference in the effect of pretreatment depending on the pretreatment solution composition. Fluorescence intensity distributions of an A549 cell spheroid impregnated with each solution of PBS- (filled circle), PBS+ (filled rhombus), PBS+Ca (*), or PBS+EDTA (open circle) for 10 minutes and then impregnated for 5 minutes in a 5 µM solution of FeRhoNoxg-1 dissolved in each solution.

### Description of Embodiments

In one aspect, the present invention relates to a method for emitting fluorescence from a target cell, comprising:
removing a calcium ion from within a cell mass or a tissue containing the target cell; and
contacting the cell mass or the tissue with a fluorescent probe which emits fluorescence when contacted with or taken up into the target cell. Preferably, the present invention is directed to a method for emitting fluorescence from the inside of the target cell mass, or a method for allowing a target cell present inside of a cell mass or a tissue to emit fluorescence, comprising the afore-mentioned steps.

In another aspect, the present invention relates to a method for determining a region where the target cells reside, comprising:
removing a calcium ion from within a cell mass or a tissue which is expected to contain the target cells;
contacting the cell mass or the tissue expected to contain the target cells with a fluorescent probe that emits fluorescence when contacted with or taken up into the target cell; and
determining a region where fluorescence emission from the fluorescent probe is observed, as the region where the target cells reside.

The present disclosure also relates to a method for determining the presence or absence of target cells, comprising:
removing a calcium ion from within a test cell mass or tissue;
contacting the test cell mass or tissue with a fluorescent probe that emits fluorescence when contacted with or taken up into the target cell; and
determining that the target cell is present when the fluorescence emission from the fluorescent probe is observed.

In the present invention, the "target cell" means a cell that emits light by contacting with or taking up the employed fluorescent probe. Thus, a target cell and an employed fluorescent probe can be appropriately selected according to a purpose. For example, when the fluorescent probe is gGlu-HMRG, the target cells are cancer cells, particularly, solid cancer cells. Examples of the "cancer" herein can include, but are not limited to, lung cancer, non-small cell lung cancer, small-cell lung cancer, non-Hodgkin's lymphoma, adrenal cortex cancer, AIDS-related cancer, AIDS-related lymphoma, childhood cerebellar astrocytoma, childhood cerebral astrocytoma, basal cell cancer, skin cancer (non-melanoma), biliary tract cancer, extrahepatic bile duct cancer, intrahepatic bile duct cancer, bladder cancer, cancer of bone or joint, osteosarcoma and malignant fibrohistiocytic tumor, brain cancer, brain tumor, brain stem glioma, cerebellar astrocytoma, glioma, cerebral astrocytoma/malignant glioma, ependymoma, medulloblastoma, supratentorial primitive neuroectodermal tumor, visual pathway and hypothalamic glioma, head and neck cancer, metastatic squamous neck cancer, bronchial adenoma/carcinoid, carcinoid tumor, nervous system lymphoma, cancer of the central nervous system, central nervous system lymphoma, neuroblastoma, childhood cancer, Sezary syndrome, extracranial germ cell tumor, extragonadal germ cell tumor, extrahepatic bile duct cancer, cancer of eye, intraocular melanoma, retinoblastoma, salivary adenocarcinoma, oral cancer, oral cavity cancer, oropharyngeal cancer, oral cancer, cancer of tongue, esophageal cancer, stomach cancer, gastrointestinal carcinoid tumor, gastrointestinal stromal tumor (GIST), small intestine cancer, colon cancer, colorectal cancer, rectal cancer, anorectal cancer, anus cancer, germ cell tumor, hepatocellular (liver) carcinoma, Hodgkin's lymphoma, voice box cancer, pharynx cancer, hypopharynx cancer, nasopharynx cancer, cancer of the sinus and the nasal passage, throat cancer, pancreatic islet cell tumor (endocrine pancreas), pancreatic cancer, parathyroid cancer, liver cancer, gallbladder cancer, appendix cancer, kidney cancer, urethral cancer, transitional cell cancer of the renal pelvis and ureter and other urinary organ cancers, squamous cell carcinoma, penis cancer, testis cancer, thymoma, thymoma and thymic cancer, thyroid gland cancer, prostate cancer, ovary cancer, epithelial ovarian cancer, ovarian low malignant potential tumor, ovarian germ cell tumor, gestational trophoblastic tumor, breast cancer, uterine cancer, uterine sarcoma, uterine cervical cancer, endometrium cancer, uterine body cancer, vaginal cancer, vulva cancer, Wilms' tumor, skin cancer (non-melanoma), skin cancer (melanoma), pheochromocytoma, Merkel cell skin cancer, mesothelioma, malignant mesothelioma, multiple endocrine neoplasia syndrome, myelodysplastic syndrome, myelodysplastic/myeloproliferative disease, pineoblastoma and pituitary gland tumor, plasmacytoma, pleuropulmonary blastoma, retinoblastoma, rhabdomyosarcoma, sarcoma, Ewing tumor, soft tissue sarcoma, soft tissue sarcoma, mycosis fungoides, and Kaposi sarcoma.

The "cell mass" herein means a massive form of an accumulated plurality of homologous or heterologous cells, and includes a tissue. The "tissue" herein means a biological structure of accumulated and three-dimensionally arranged homologous or heterologous cells. For example, tissue includes an epithelial tissue, a connective tissue, a muscle tissue, and a nerve tissue. The "cell mass or tissue containing the target cell" may contain cells or tissues other than the target cell as long as the cell mass or the tissue contains the target cell. The "cell mass or tissue expected to contain the target cell" is not necessarily required to contain the target cell, but can be a cell mass or a tissue that has possibility to contain the target cell, and may contain cells or tissues other than the target cell. The "target cell mass" means a cell mass composed mainly of the target cell.

The target cell emits the fluorescence by contacting with the fluorescent probe at a surface or taking up the fluorescent probe into the cell.

In the invention, the fluorescent probe emits fluorescence when reacted with an enzyme or iron ions. The enzyme is not particularly limited as long as it is a protein capable of catalyzing particular reaction. Examples thereof can include glycosidase, protease, and peptidase (γ-glutamyl transpeptidase, etc.). The enzyme may be present on cell membrane surface or intracellularly.

The fluorescent probe may be, for example, a substance that is hydrophilized not to be taken up into a cell. Specific examples of the fluorescent probe can include gGlu-HMRG, Leu-HMRG, EP-HMRG and FeRhoNox-1. Alternatively, the fluorescent probe may be in a DDS form which is, for example, embedded in a vehicle such as a liposome.

In the invention the calcium ions are removed by impregnating the cell mass or the tissue containing the target cell in a buffer containing a metal ion chelating agent or a buffer solution containing no calcium. As the metal ion chelating agent, a divalent metal ion chelating agent such as ethylenediaminetetraacetic acid (EDTA), glycol ether diamine tetraacetic acid (EGTA), or citric acid can be used. Examples of the buffer solution include phosphate-buffered saline (PBS), phosphate buffer solutions, sodium citrate buffer solutions, citrate-phosphate buffer solutions, Tris-hydrochloride buffer solutions (Tris-HCl), MES, PIPES, HEPES, ADA, ACES, MOPSO, BES, MOPS, TES, DIPSO, TAPSO, POPSO, HEPPSO, EPPS, bicine, TAPS, and tricine. For example, the impregnation can be performed for 30 seconds to 1 hour, 30 seconds to 30 minutes, 1 to 20 minutes, 3 to 10 minutes, 5 to 15 minutes, or 10 to 20 minutes. The impregnation can be performed at pH 7 to 8. The calcium ion does not need to be removed 100%, and is enough removed when cell-cell adhesion is loosen to allow the fluorescent probe to reach a site of interest in the cell mass or the tissue. Particularly, when the fluorescent probe generates a fluorescent substance by reaction with a substance on cell surface, the removal of the calcium ion is desirably performed under conditions that do not wash out the substance contained in the surface of the cell mass or the tissue and the fluorescent substance formed through the reaction.

The contact of the fluorescent probe with the cell mass or the tissue may be performed by impregnating the cell mass or the tissue with a liquid containing the fluorescent probe, or may be performed by dropping or spraying a liquid containing the fluorescent probe to the cell mass or the tissue or a site comprising the cell mass or the tissue. The liquid containing the fluorescent probe is preferably a buffer solution, more preferably a buffer solution capable of removing calcium ions from the cell mass or the tissue. For example, the fluorescent probe may be used as a form contained in a buffer containing a chelating agent or PBS containing no calcium.

The fluorescence emission from the target cell can be measured and observed by appropriately detecting light emission at a wavelength according to the employed fluorescent probe using an apparatus and a method well known to those skilled in the art. Preferably, the fluorescence emission from the target cell have intensity that can distinguish the target cell from non-target cells. Further, the fluorescence emission from the target cell may have intensity that can visually distinguish the target cell from non-target cells.

The method of the present invention is particularly useful in the case where a tumor cell or a cancer cell is present inside of a cell mass and thus cannot be detected by only contacting with a fluorescent probe. By using the method of the present invention, the tumor cell or the cancer cell which is present inside of a cell mass can emit light, and thereby the presence of tumor or cancer present inside of a cell mass or a tissue can be detected more reliably.

Accordingly, in one aspect, the target cell is a cancer cell or a tumor cell. The present invention relates to a method for discriminating a cancer cell or a tumor cell from non-cancer cells or non-tumor cells, comprising allowing the cancer cell or the tumor cell to emit fluorescence by the aforementioned method, and determining a cell emitting fluorescence as the cancer cell or the tumor cell and determining cells emitting no fluorescence as non-cancer cells or non-tumor cells, thereby discriminating the cancer cell or the tumor cell from non-cancer cells or non-tumor cells. Such discrimination of a cancer cell or a tumor cell from non-cancer cells or non-tumor cells enables reliable removal of cancer cells or tumor cells during surgery, for example, and can enhance the diagnostic accuracy of cancer or tumor diagnosis.

In another aspect, the present invention relates to a method for diagnosing or monitoring cancer or tumor in a subject, or a method for providing information therefor, comprising removing calcium ions from within a cell mass sample or a tissue sample obtained from the subject; contacting the sample with a fluorescent probe as described above that emits fluorescence when contacted with or taken up into a cancer cell or a tumor cell; and when a cell emitting fluorescence is present in the sample, diagnosing the subject as having the cancer or the tumor or judging the cancer or the tumor existing in the subject (or judging the subject being poor progress or poor prognosis), or providing information thereof. In this method, examples of the fluorescent probe that emits fluorescence by contacting with a cancer cell or a tumor cell or takin up into a cancer cell or a tumor cell include gGlu-HMRG. The "monitoring method" herein includes the monitoring of the presence or absence of cancer or tumor in a patient who may have cancer or tumor, and the monitoring whether a progress after operation or treatment of a cancer or tumor patient is good or poor, or whether a prognosis of a cancer or tumor patient is good or poor.

The method of the present invention is performed *ex vivo.*

Hereinafter, the present invention will be described more specifically with reference to Examples. However, the present invention is not limited by these Examples.

### Examples

### (Example 1) Clinical specimen

Lymph nodes including sentinel lymph node excised for the purpose of pathological diagnosis during breast cancer surgery were divided and transported from the operating room to an inspection room in a state wrapped in gauze wetted with PBS. A reagent and PBS (140 mM NaCl, 2.7 mM KCl, 10 mM PO₄³⁻, pH = 7.4) at room temperature were used. A PBS impregnated sample was impregnated with a container where PBS was placed, and continuously impregnated for 1 minute, 5 minutes, 10 minutes, or an appropriate course of time from 1 minute to 30 minutes. A PBS non-impregnated sample was stored in state wrapped in gauze wetted with PBS to prevent drying out, instead of being impregnated with PBS, and used in fluorescence measurement. The lymph nodes were carefully taken out with tweezers and put on a Petri dish, and visible images of autofluorescence were taken in a fluorescence measuring machine. 50 µM gGlu-HMRG dissolved in PBS was added to the Petri dish where the lymph nodes were placed, until the lymph nodes completely soaked therein. The fluorescence measurement was continued. The pathological diagnosis of the specimens used in this test was also conducted and checked against final results about whether cancer metastasized to each lymph node.

Increase in fluorescence intensity in the lymph nodes 10 minutes after the start of measurement is shown in Figure 1. The fluorescence intensity was indicated by a ratio to the fluorescence intensity of a fluorescence intensity reference plate having a constant fluorescence intensity (measured fluorescence intensity / fluorescence intensity of the fluorescence intensity reference plate). Among a total of 25 specimens, 5 specimens confirmed to have no cancer metastasis in pathological diagnosis also exhibited little fluorescence increase. Among 20 cases confirmed to have cancer, specimens impregnated with PBS were observed to emit strong fluorescence, regardless of their impregnating times. On the other hand, some specimens without impregnation with PBS were observed to emit only weak fluorescence, and such cases were observed to have difficulty in discriminating from specimens free from cancer cells. These results demonstrated that impregnated with PBS before detection of cancer with a fluorescent reagent enhances the accuracy of diagnosis using gGlu-HMRG.

### (Example 2) Change in fluorescence intensity of gGlu-HMRG in spheroid formed from cancer-derived cultured cell

HeLa cells were inoculated at 10⁵ cells/well and cultured for 4 to 6 days. The obtained spheroid was put on a glass bottom dish and cultured for about 3 hours. Then, the culture solution was removed, and the spheroid was impregnated with PBS(-) for 10 minutes. A control was washed with HBSS without being impregnated with PBS(-). A 1 µM solution of gGlu-HMRG dissolved in PBS(-) or HBSS was added to the spheroid impregnated with PBS(-) or the control spheroid. Each spheroid immediately (0 minutes), 10 minutes, 20 minutes, 30 minutes, and 60 minutes after addition of the gGlu-HMRG solution was photographed under a fluorescence microscope (Leica DMI 6000 CS, lens: 10×, filter Leica L5, excitation wavelength: 460 to 500 nm, fluorescence wavelength: 512 to 542 nm). Transmitted light images of the spheroid were taken 10 minutes after impregnated with PBS(-).

The results of fluorescence observation are shown in Figure 2. A slower increase of fluorescence in the central portion of the spheroid was observed for HBSS (physiological buffer solution more similar to blood) compared with PBS(-). This indicated that solution composition or preliminary impregnating treatment is important for the reaction or penetration rate of a reagent. Furthermore, weakened adhesion between cells by impregnating with PBS(-) was observed in the transmitted light images (Figure 3).

### (Example 3) Quantification of degrees of gGlu-HMRG reaction and penetration using cancer-derived cultured cell spheroid

HeLa cells (human uterine cervical cancer cell line) or A549 cells (human alveolar basal epithelial adenocarcinoma cell line) were inoculated at 1 × 10⁴ cells to Nunclon Sphera and cultured for 7 to 14 days. Each obtained spheroid was allowed to adhere to a poly-L-lysine-coated glass bottom dish over approximately 24 hours. The medium was replaced with a buffer, and the spheroid was incubated at room temperature for 10 minutes (impregnation, rinse). The buffer in the dish was replaced with each buffer containing gGlu-HMRG dissolved to attain a final concentration of 0.1 µM (A549 cells) or 1 µM (HeLa cells), followed by observation under a fluorescence microscope (Leica DMI 6000 CS, lens: 10×, filter Leica L5, ex. 460 to 500 nm, em. 512 to 542 nm). An area (ROI) having the distance from surface shown in Figure 4 was captured with ImageJ, and increase in the average luminance between the start of measurement and 5 minutes later was analyzed. Fluorescence was also observed by impregnating with saline and addition of gGlu-HMRG dissolved in PBS(-).

The buffers used and their composition are as follows.
PBS(-): 140 mM NaCl, 2.7 mM KCl, 10 mM PO₄³⁻ (pH = 7.4)
PBS(+): PBS(-) described above as well as 0.90 mM CaCl₂, 0.33 mM MgCl₂ (pH = 7.4)
PBS-Ca: PBS(-) described above as well as 0.90 mM CaCl₂ (pH = 7.4)
PBS-Mg: PBS(-) described above as well as 0.33 mM MgCl₂ (pH = 7.4)
PBS-EDTA: PBS(-) described above as well as 0.2 mM EDTA (pH = 7.4)
Saline: 154 mM NaCl
HBSS(+): HBSS-Hank's Balanced Salt Solution (Thermo Fisher Scientific Inc, #14025076)

The results about PBS(-) and HBSS(+) obtained using HeLa cells are shown in Figure 5. When a spheroid formed from the HeLa cells was reacted with gGlu-HMRG dissolved in HBSS similar to body fluid, without being washed with a buffer, the increase of fluorescence was slowed with the increasing distance from surface. In contrast, the increase of fluorescence was mora rapid by impregnating with gGlu-HMRG dissolved in PBS(-) after impregnation with PBS(-) for 10 minutes.

The results about PBS(-), PBS(+), PBS-Ca, and PBS-Mg obtained using A549 cells are shown in Figure 6. PBS(-) impregnating treatment compared with impregnation with PBS(+) accelerated the increase rate of fluorescence intensity. Substantially the same results as those of PBS(-) were obtained in PBS-Mg of PBS(-) supplemented with only Mg²⁺. On the other hand, use of PBS-Ca of PBS supplemented with Ca²⁺ less increased fluorescence, as in PBS(+). These results suggested that the presence or absence of calcium ions is important.

The results about addition of gGlu-HMRG dissolved in PBS(-), PBS(+), saline, saline+PBS(-), obtained using A549 cells are shown in Figure 7. The increase of fluorescence intensity was slow for saline. The effect of stabilizing pH by a phosphate buffer contained in PBS is also considered to be important.

### (Example 4) Evaluation of influence of chelating agent on degrees of gGlu-HMRG reaction and penetration using cancer-derived cultured cell spheroid

To evaluate the influence of divalent ion (calcium, etc.)-dependent adhesion, fluorescence was observed under the same conditions as in Example 3 except that the following buffers were used.
(1) No pretreatment (no rinse) and impregnation with a solution of gGlu-HMRG dissolved in PBS(-)
(2) Pretreatment by impregnating with PBS(-)
(3) Pretreatment by impregnating with PBS(+)
(4) Pretreatment by impregnating with PBS(-)+0.2 mM EDTA

The results obtained using HeLa cells are shown in Figure 8. Impregnating with PBS(-) containing no divalent ions (Ca²⁺, Mg²⁺), compared with no impregnation or impregnation with PBS(+) containing a divalent ion, accelerated the increase of fluorescence at a location distant from surface. The addition of a divalent ion chelator (EDTA) further accelerated the increase of fluorescence.

The results obtained using A549 cells are shown in Figure 9. The A549 cells exhibited a tendency similar to that of the HeLa cells. PBS(+) which is PBS(-) supplemented with a divalent ion (Ca²⁺, Mg²⁺) had an increase rate of fluorescence almost equivalent to that of no impregnation. On the other hand, the addition of 0.2 mM EDTA chelating a divalent ion caused larger increase of fluorescence intensity at a site deeper from surface even compared with PBS(-).

### (Example 5) Influence of pretreatment when different fluorescent probe is used

Fluorescence was measured by the same method as in Example 3 except that a 5 µM FeRhoNox-1 probe was used instead of gGlu-HMRG as the fluorescent probe. The cells used were only A549 cells.

The results are shown in Figure 10. The same tendency as that obtained using gGlu-HMRG as a fluorescent probe was observed in the FeRhoNox-1 probe. This indicated that the removal of calcium promotes the penetration of another fluorescent probe into a cell mass.

Thus, impregnation with PBS(-) in advance accelerated the increase of fluorescence from gGlu-HMRG in the central portion of a spheroid. This effect was rarely seen in a solution containing calcium, such as PBS(+) or PBS-Ca²⁺. This effect was rather more potentiated by active removal of calcium ions with PBS-EDTA. The cell images suggest that impregnation in PBS(-) inhibits calcium-dependent adhesion and creates a gap between cells, through which a reagent easily penetrates. Similarly, no clear effect was seen in saline containing no calcium. This phenomenon was observed not only in HeLa cells but also in A549 cells and is considered to be independent on a particular cell type. Also, this phenomenon was observed not only in gGlu-HMRG but also in FeRhoNox-1, indicating applicability when a feature of cells is to be detected with a functional fluorescent probe.

### (Example 6) Influence of pH: Influence of pretreatment when different fluorescent probe is used

Cells cultured on glass surface were reacted with gGlu-HMRG, and fluorescence intensity was compared. HeLa cells were inoculated at the same density to chambers of a 4-chamber glass bottom dish. The cells were cultured in DMEM+8% FBS, P.S. under conditions of 37°C and 5% CO₂ for approximately 8 hours. The cells were rinsed three times with PBS(-) adjusted to pH 6.5, 7.3, or 7.8, which was then replaced with each solution supplemented with 2 µM gGlu-HMRG. The cells were stained at 37°C under 5% CO₂ for 20 minutes and observed under a fluorescence microscope under the same conditions as above without the solution replaced. 100 ms exposure, gain 5 Int 5, ND1. Fluorescence intensity from 5 cells in fluorescence images, and background fluorescence intensity were measured with reference to superimposed images with DIC, and the mean and standard deviation were plotted. The fluorescence intensity of gGlu-HMRG and the fluorescence intensity of HMRG (fluorescent substance after reaction with GGT) were measured at each pH.

Fluorescence intensity had pH dependency, and lower pH decreased a signal and increased a background (not shown). The pH of 7.3 or higher increased signal intensity. The fluorescence intensity of gGlu-HMRG and the fluorescence intensity of HMRG (fluorescent substance after reaction with GGT) were measured at each pH. As a result, the fluorescence intensity was decreased with the elevation of pH, indicating that a pH condition of 6 to 8 is preferred with pH 6 to 7.5 more preferred (not shown). These results indicated that pH influences the measurement of fluorescence intensity.

## Claims

1. A method for emitting fluorescence that can distinguish a target cell from non-target cells, comprising:
removing a calcium ion from within a cell mass or a tissue containing the target cell,
contacting the cell mass or the tissue with a fluorescent probe which emits fluorescence when contacted with or taken up into the target cell, and
enabling the cell mass or the tissue from which the calcium ion was removed and which was contacted with the fluorescent probe to emit fluorescence,
wherein the fluorescent probe that emits fluorescence when contacted with or taken up into the target cell is a fluorescent probe that emits fluorescence when reacted with an enzyme or iron ions contained in the target cell,
wherein the removal of the calcium ion is performed by impregnating the cell mass or the tissue containing the target cell with a buffer solution containing a metal ion chelating agent or a buffer solution containing no calcium,
wherein the method is performed *ex* vivo.

2. The method of claim 1, which is a method for emitting fluorescence from the inside of the target cell mass, or a method for allowing a target cell present inside of the cell mass or the tissue to emit fluorescence.

3. A method for determining a region of presence of a target cell, comprising:
removing a calcium ion from within a cell mass or a tissue which is expected to contain the target cell;
contacting the cell mass or the tissue expected to contain the target cell with a fluorescent probe that emits fluorescence when contacted with or taken up into the target cell; and
determining a region where fluorescence emission from the fluorescent probe is observed, as the region of presence of the target cell,
wherein the fluorescent probe that emits fluorescence when contacted with or taken up into the target cell is a fluorescent probe that emits fluorescence when reacted with an enzyme or iron ions contained in the target cell,
wherein the removal of the calcium ion is performed by impregnating the cell mass or the tissue containing the target cell with a buffer solution containing a metal ion chelating agent or a buffer solution containing no calcium,
wherein the method is performed *ex vivo.*

4. The method of claim 1, wherein the enzyme is glycosidase, protease, or peptidase.

5. The method of claim 4, wherein the enzyme is an enzyme present on cell membrane surface.

6. The method of any one of the preceding claims, wherein the impregnation is performed for 30 seconds to 30 minutes.

7. The method of claim 1, wherein the fluorescent probe emits fluorescence when contacted with or taken up into the target cell,
wherein the target cell is a cancer cell or a tumor cell.

8. The method of claim 7, wherein the cancer is solid cancer.

9. The method of any one of the preceding claims, wherein the fluorescent probe is gGlu-HMRG, Leu-HMRG, EP-HMRG or FeRhoNox-1.

10. A method for diagnosing cancer or tumor, comprising:
removing a calcium ion from within a cell mass sample or a tissue sample obtained from a subject;
contacting the sample with a fluorescent probe that emits fluorescence when contacted with or taken up into a cancer cell or a tumor cell; and
diagnosing the subject as having cancer or tumor when a cell emitting fluorescence is present in the sample,
wherein the fluorescent probe that emits fluorescence when contacted with or taken up into the target cell is a fluorescent probe that emits fluorescence when reacted with an enzyme or iron ions contained in the target cell,
wherein the removal of the calcium ion is performed by impregnating the cell mass or the tissue containing the target cell with a buffer solution containing a metal ion chelating agent or a buffer solution containing no calcium,
wherein the method is performed *ex vivo.*

11. The method of claim 10, wherein the method comprises providing image data on the sample from which the calcium ion was removed and which was contacted with the fluorescent probe.

12. The method of claim 10 or 11, wherein the impregnation is performed for 30 seconds to 30 minutes.

## Patentansprüche

1. Verfahren zum Emittieren von Fluoreszenz, die eine Zielzelle von Nicht-Zielzellen unterscheiden kann, umfassend:
Entfernen eines Calciumions aus innerhalb einer Zellmasse oder eines Gewebes, die/das die Zielzelle enthält,
Inberührungbringen der Zellmasse oder des Gewebes mit einer fluoreszierenden Sonde, die Fluoreszenz emittiert, wenn sie mit der Zielzelle in Berührung kommt oder von dieser aufgenommen wird, und
Aktivieren der Zellmasse oder des Gewebes, aus der/dem das Calciumion entfernt wurde und die/der mit der fluoreszierenden Sonde in Berührung gebracht wurde, um Fluoreszenz zu emittieren,
wobei die Fluoreszenzsonde, die Fluoreszenz emittiert, wenn sie mit der Zielzelle in Berührung kommt oder in diese aufgenommen wird, eine Fluoreszenzsonde ist, die Fluoreszenz emittiert, wenn sie mit einem Enzym oder Eisenionen reagiert, das/die in der Zielzelle enthalten ist/sind,
wobei die Entfernung des Calciumions durch Imprägnieren der Zellmasse oder des Gewebes, die/das die Zielzelle enthält, mit einer Pufferlösung, die einen Metallionenchelatbildner enthält, oder einer Pufferlösung, die kein Calcium enthält, durchgeführt wird,
wobei das Verfahren *ex vivo* durchgeführt wird.

2. Verfahren nach Anspruch 1, wobei es ein Verfahren zum Emittieren von Fluoreszenz aus dem Inneren der Zielzellmasse oder ein Verfahren ist, bei dem eine im Innern der Zellmasse oder dem Gewebe vorhandene Zielzelle Fluoreszenz emittieren kann.

3. Verfahren zum Bestimmen eines Vorhandenseinbereichs einer Zielzelle, umfassend:
Entfernen eines Calciumions aus innerhalb einer Zellmasse oder eines Gewebes, die/das die Zielzelle enthält,
Inberührungbringen der Zellmasse oder des Gewebes, von der/dem erwartet wird, dass sie/es die Zielzelle enthält, mit einer fluoreszierenden Sonde, die Fluoreszenz emittiert, wenn sie mit der Zielzelle in Berührung kommt oder von dieser aufgenommen wird; und
Bestimmen eines Bereichs, in dem die Fluoreszenzemission von der Fluoreszenzsonde beobachtet wird, als den Vorhandenseinbereich der Zielzelle,
wobei die Fluoreszenzsonde, die Fluoreszenz emittiert, wenn sie mit der Zielzelle in Berührung kommt oder in diese aufgenommen wird, eine Fluoreszenzsonde ist, die Fluoreszenz emittiert, wenn sie mit einem Enzym oder Eisenionen reagiert, das/die in der Zielzelle enthalten ist/sind,
wobei die Entfernung des Calciumions durch Imprägnieren der Zellmasse oder des Gewebes, die/das die Zielzelle enthält, mit einer Pufferlösung, die einen Metallionenchelatbildner enthält, oder einer Pufferlösung, die kein Calcium enthält, durchgeführt wird,
wobei das Verfahren *ex vivo* durchgeführt wird.

4. Verfahren nach Anspruch 1, wobei das Enzym eine Glycosidase, Protease oder Peptidase ist.

5. Verfahren nach Anspruch 4, wobei das Enzym ein Enzym ist, das auf der Oberfläche der Zellmembran vorhanden ist.

6. Verfahren nach einem der vorhergehenden Ansprüche, wobei die Imprägnierung für 30 Sekunden bis 30 Minuten durchgeführt wird.

7. Verfahren nach Anspruch 1, wobei die fluoreszierende Sonde Fluoreszenz emittiert, wenn sie mit der Zielzelle in Berührung kommt oder von dieser aufgenommen wird,
wobei die Zielzelle eine Krebszelle oder eine Tumorzelle ist.

8. Verfahren nach Anspruch 7, wobei der Krebs ein fester Krebs ist.

9. Verfahren nach einem der vorhergehenden Ansprüche, wobei die Fluoreszenzsonde gGlu-HMRG, Leu-HMRG, EP-HMRG oder FeRhoNox-1 ist.

10. Verfahren zum Diagnostizieren von Krebs oder eines Tumors, umfassend:
Entfernen eines Calciumions aus innerhalb einer Zellmassenprobe oder einer Gewebeprobe, die von einem Subjekt erhalten wird;
Inberührungbringen der Probe mit einer fluoreszierenden Sonde, die Fluoreszenz emittiert, wenn sie mit einer Krebszelle oder einer Tumorzelle in Berührung kommt oder von dieser aufgenommen wird; und
Diagnostizieren des Subjekts mit Krebs oder einem Tumor, wenn eine Zelle, die Fluoreszenz emittiert, in der Probe vorhanden ist,
wobei die Fluoreszenzsonde, die Fluoreszenz emittiert, wenn sie mit der Zielzelle in Berührung kommt oder in diese aufgenommen wird, eine Fluoreszenzsonde ist, die Fluoreszenz emittiert, wenn sie mit einem Enzym oder Eisenionen reagiert, das/die in der Zielzelle enthalten ist/sind,
wobei die Entfernung des Calciumions durch Imprägnieren der Zellmasse oder des Gewebes, die/das die Zielzelle enthält, mit einer Pufferlösung, die einen Metallionenchelatbildner enthält, oder einer Pufferlösung, die kein Calcium enthält, durchgeführt wird,
wobei das Verfahren *ex vivo* durchgeführt wird.

11. Verfahren nach Anspruch 10, wobei das Verfahren das Bereitstellen von Bilddaten auf der Probe, von der das Calciumion entfernt wurde und die mit der Fluoreszenzsonde in Berührung gebracht wurde, umfasst.

12. Verfahren nach Anspruch 10 oder 11, wobei die Imprägnierung für 30 Sekunden bis 30 Minuten durchgeführt wird.

## Revendications

1. Procédé d'émission de fluorescence permettant de distinguer une cellule cible de cellules non cibles, comprenant :
l'élimination d'un ion calcium du sein d'une masse cellulaire ou d'un tissu contenant la cellule cible,
la mise en contact de la masse cellulaire ou du tissu avec une sonde fluorescente qui émet une fluorescence lorsqu'elle est en contact avec la cellule cible ou absorbée dans celle-ci, et
le fait de laisser la masse cellulaire ou le tissu dont l'ion calcium a été éliminé et qui a été mis en contact avec la sonde fluorescente, d'émettre une fluorescence,
dans lequel la sonde fluorescente qui émet une fluorescence lorsqu'elle est mise en contact avec la cellule cible ou est absorbée dans celle-ci est une sonde fluorescente qui émet une fluorescence lorsqu'elle est mise à réagir avec une enzyme ou des ions fer contenus dans la cellule cible,
dans lequel l'élimination de l'ion calcium est réalisée par imprégnation de la masse cellulaire ou du tissu contenant la cellule cible avec une solution tampon contenant un agent chélateur d'ions métalliques ou une solution tampon ne contenant pas de calcium,
le procédé étant réalisé *ex vivo.*

2. Procédé selon la revendication 1, qui est un procédé d'émission de fluorescence à partir de l'intérieur de la masse cellulaire cible, ou un procédé permettant à une cellule cible présente à l'intérieur de la masse cellulaire ou du tissu d'émettre une fluorescence.

3. Procédé de détermination d'une région de présence d'une cellule cible, comprenant :
l'élimination d'un ion calcium du sein d'une masse cellulaire ou d'un tissu censé contenir la cellule cible ;
la mise en contact de la masse cellulaire ou du tissu censé contenir la cellule cible avec une sonde fluorescente qui émet une fluorescence lorsqu'elle est mise en contact avec la cellule cible ou absorbée dans celle-ci ; et
la détermination d'une région où l'émission de fluorescence à partir de la sonde fluorescente est observée, en tant que région de présence de la cellule cible,
dans lequel la sonde fluorescente qui émet une fluorescence lorsqu'elle est mise en contact avec la cellule cible ou est absorbée dans celle-ci est une sonde fluorescente qui émet une fluorescence lorsqu'elle mise à réagir avec une enzyme ou des ions fer contenus dans la cellule cible,
dans lequel l'élimination de l'ion calcium est réalisée par imprégnation de la masse cellulaire ou du tissu contenant la cellule cible avec une solution tampon contenant un agent chélateur d'ions métalliques ou une solution tampon ne contenant pas de calcium,
le procédé étant réalisé *ex vivo.*

4. Procédé selon la revendication 1, dans lequel l'enzyme est une glycosidase, une protéase, ou une peptidase.

5. Procédé selon la revendication 4, dans lequel l'enzyme est une enzyme présente sur la surface de membrane cellulaire.

6. Procédé selon l'une quelconque des revendications précédentes, dans lequel l'imprégnation est réalisée pendant 30 secondes à 30 minutes.

7. Procédé selon la revendication 1, dans lequel la sonde fluorescente émet une fluorescence lorsqu'elle est mise en contact avec la cellule cible ou absorbée dans celle-ci,
dans lequel la cellule cible est une cellule cancéreuse ou une cellule tumorale.

8. Procédé selon la revendication 7, dans lequel le cancer est un cancer solide.

9. Procédé selon l'une quelconque des revendications précédentes, dans lequel la sonde fluorescente est gGlu-HMRG, Leu-HMRG, EP-HMRG ou FeRhoNox-1.

10. Procédé de diagnostic d'un cancer ou d'une tumeur, comprenant :
l'élimination d'un ion calcium du sein d'un échantillon de masse cellulaire ou d'un échantillon de tissu prélevé sur un sujet ;
la mise en contact de l'échantillon avec une sonde fluorescente qui émet une fluorescence lorsqu'elle est mise en contact avec une cellule cancéreuse ou une cellule tumorale ou absorbée dans celle-ci ; et
le diagnostic d'un cancer ou d'une tumeur chez le sujet lorsqu'une cellule émettant une fluorescence est présente dans l'échantillon,
dans lequel la sonde fluorescente qui émet une fluorescence lorsqu'elle est mise en contact avec la cellule cible ou absorbée dans celle-ci est une sonde fluorescente qui émet une fluorescence lorsqu'elle est mise à réagir avec une enzyme ou des ions fer contenus dans la cellule cible,
dans lequel l'élimination de l'ion calcium est réalisée par imprégnation de la masse cellulaire ou du tissu contenant la cellule cible avec une solution tampon contenant un agent chélateur d'ions métalliques ou une solution tampon ne contenant pas de calcium,
le procédé étant réalisé *ex vivo.*

11. Procédé selon la revendication 10, le procédé comprenant la fourniture de données d'image sur l'échantillon dont l'ion calcium a été éliminé et qui a été mis en contact avec la sonde fluorescente.

12. Procédé selon la revendication 10 ou 11, dans lequel l'imprégnation est réalisée pendant 30 secondes à 30 minutes.
